# EUROPEAN PATENT APPLICATION

(11) **EP 4 609 861 A1**
(43) Date of publication of application: **03.09.2025**
(21) Application number: 23881879.3
(22) Date of filing: 25.10.2023
(51) Int. Cl.: A61K 31/4155, A61P 7/04, A61P 7/00

(54) **USE OF HEROMBOPAG OR PHARMACEUTICALLY ACCEPTABLE SALT THEREOF IN TREATMENT OF TUMOR CHEMOTHERAPY-INDUCED THROMBOCYTOPENIA**

(30) Priority: 25.10.2022 CN 202211313829; 22.03.2023 CN 202310288757
(71) Applicant: Jiangsu Hengrui Pharmaceuticals Co., Ltd., Lianyungang, Jiangsu 222047 (CN)
(72) Inventor: WANG, Quanren, Lianyungang, Jiangsu 222047 (CN); LI, Weixia, Lianyungang, Jiangsu 222047 (CN); TANG, Qian, Lianyungang, Jiangsu 222047 (CN)
(74) Representative: dompatent von Kreisler Selting Werner - Partnerschaft von Patent- und Rechtsanwälten mbB
(86) International application number: PCT/CN2023/126452
(87) International publication number: WO 2024/088302

(57) **Abstract**

The present disclosure relates to a use of Herombopag or a pharmaceutically acceptable salt thereof in treatment of tumor chemotherapy-induced thrombocytopenia. Specifically, the present disclosure relates to a disease treatment method, comprising: administering to a patient Herombopag or a pharmaceutically acceptable salt thereof, which can significantly increase the blood platelet count of a subject.

## Description

The present application claims priority to Chinese Patent Application No. 2022113138298, filed on October 25, 2022, and Chinese Patent Application No. 2023102887574, filed on March 22, 2023, which are incorporated herein by reference in their entirety.

### TECHNICAL FIELD

The present disclosure relates to use of herombopag or a pharmaceutically acceptable salt thereof in the treatment of thrombocytopenia caused by chemotherapy for malignant tumors.

### BACKGROUND

Thrombocytopenia is manifested in a platelet count in peripheral blood that falls below the normal level and thus leads to varying degrees of bleeding or bleeding risk. Severe thrombocytopenia is associated with a higher mortality rate. Based on its etiology, thrombocytopenia can be classified into several categories: decreased platelet production (e.g., leukemia, bone metastases of malignant tumors, chemotherapeutic drugs, radiation, aplastic anemia, and megaloblastic anemia), increased destruction or excessive consumption (including immune destruction, such as immune thrombocytopenia [ITP] and systemic lupus erythematosus, and non-immune destruction, such as thrombotic thrombocytopenic purpura and vasculitis), abnormal distribution (splenomegaly due to various causes), and platelet loss (e.g., bleeding and hemodialysis). Among these, chemotherapy-induced thrombocytopenia (CIT) is a clinically common dose-limiting toxicity of chemotherapeutic drugs. It results from the inhibition of bone marrow, particularly megakaryocytes, by antitumor chemotherapeutic drugs, which leads to insufficient production and excessive destruction of platelets, causing a reduction in peripheral blood platelet counts (<100 × 109/L). In CIT, the timing of the lowest platelet count and the degree of platelet reduction are associated with the chemotherapeutic drug used, the dosage, whether combination therapy is employed, individual differences between patients, and the number of chemotherapy cycles. The incidence of CIT is approximately 10%-36% in solid tumors and as high as 75% in hematologic tumors. The direct consequence of CIT is bleeding, such as mucocutaneous bleeding, epistaxis, gum bleeding, hemoptysis, hematemesis, hematuria, heavy menstrual bleeding in women, and intracranial hemorrhage. Indirectly, CIT can cause an increase in the need for platelet transfusions, a reduction in chemotherapeutic drug doses, a delay in chemotherapy, or even discontinuation of chemotherapy, thereby impacting the chemotherapy schedule and efficacy and even increasing the mortality rate. In addition, CIT can prolong hospital stays, raise medical costs, and impact long-term clinical outcomes. Severe thrombocytopenia is associated with poor clinical prognosis, including an increased risk of bleeding and an increased mortality rate.

Chemotherapy regimens containing platinum-based formulations and gemcitabine (used for treating lung cancer, bladder cancer, ovarian cancer, cervical cancer, gastrointestinal tumors, etc.) easily cause CIT, especially grade 3/4 CIT. CIT is typically manifested in a platelet count that begins to decline on day 3-7 after chemotherapy, reaches the lowest point on day 14 post-chemotherapy, then gradually rises, and recovers to the baseline level on day 28-35. The incidence of CIT gradually increases with the number of chemotherapy cycles. A large-scale, 7-year (2000-2007) epidemiological study of outpatient cancer patients in the United States analyzed 43,995 patients with evaluable platelet counts. In the beginning, 11.1% of patients had thrombocytopenia. After initial chemotherapy, the incidence of thrombocytopenia varied by regimen, and it was 64.2% with gemcitabine-based regimens, 55.4% with platinum-based regimens, 37.8% with anthracycline-based regimens, and 21.9% with paclitaxel-based regimens. The incidence of grade 3/4 thrombocytopenia was 11.2% with gemcitabine-based regimens, 10.6% with platinum-based regimens, 5.2% with anthracycline-based regimens, and 1.9% with paclitaxel-based regimens. Among various types of tumors, the incidence of thrombocytopenia was 61.7% in colorectal cancer, 50.5% in NSCLC, 45.6% in ovarian cancer, and 37.6% in breast cancer. The highest incidence of grade 3/4 thrombocytopenia was observed in NSCLC, and it was 10.7%. Among all tumor patients undergoing chemotherapy, the platelet transfusion rate was 2.5%, and the chemotherapy delay rate was 22.7%, with an average delay of 17 days. Elias A. et al. also reported that 48% of patients with advanced sarcomas receiving chemotherapy with doxorubicin, ifosfamide, and dacarbazine developed grade 3/4 thrombocytopenia. Over 50% of NSCLC patients receiving chemotherapy with carboplatin, ifosfamide, and etoposide developed CIT. In ovarian cancer patients receiving chemotherapy with paclitaxel, ifosfamide, and cisplatin, the incidence of CIT was 24%-33%.

At present, there are no clinical oral thrombopoietic drugs that can rapidly increase platelet counts and, while maintaining relative chemotherapy dose intensity, remain safe and effective.

### SUMMARY

The present disclosure provides use of herombopag or a pharmaceutically acceptable salt thereof in the preparation of a medicament for treating chemotherapy-induced thrombocytopenia, wherein the herombopag or the pharmaceutically acceptable salt thereof is a unit-dose formulation comprising 7.5 mg of herombopag.

In some embodiments, a patient with chemotherapy-induced thrombocytopenia has a platelet count of <75 × 10⁹/L.

In some embodiments, the patient with chemotherapy-induced thrombocytopenia experiences a delay of ≥1 week in chemotherapy relative to an expected time due to thrombocytopenia and has a platelet count of <75 × 10⁹/L.

In some embodiments, a chemotherapy regimen for the patient with chemotherapy-induced thrombocytopenia is a combination therapy regimen comprising a platinum-based drug, and the platinum-based drug includes, but is not limited to, carboplatin, nedaplatin, cisplatin, lobaplatin, Eloxatin, oxaliplatin, etc.

In another aspect, in some embodiments, after the patient is treated with herombopag or the pharmaceutically acceptable salt thereof, the patient has a platelet count that is greater than or equal to 100 × 10⁹/L and less than 200 × 10⁹/L, and the treatment is maintained at an original dose.

In some embodiments, after the patient is treated with herombopag or the pharmaceutically acceptable salt thereof for 14 days, the patient has a platelet count that is greater than or equal to 100 × 10⁹/L and less than 200 × 10⁹/L, and the treatment is maintained at an original dose.

In some other embodiments, after the patient is treated with herombopag or the pharmaceutically acceptable salt thereof, the patient has a platelet count that is greater than 400 × 10⁹/L, and the treatment is suspended, and when the platelet count decreases to <200 × 10⁹/L, the treatment is resumed, and a daily dose is decreased by 2.5 mg; if a lowest dose used at this time is 2.5 mg/day, the treatment continues to be stopped.

In some embodiments, after the patient is treated with herombopag or the pharmaceutically acceptable salt thereof for 14 days, the patient has a platelet count that is greater than 400 × 10⁹/L, and the treatment is suspended, and when the platelet count decreases to <200 × 10⁹/L, the treatment is resumed, and a daily dose is decreased by 2.5 mg; if a lowest dose used at this time is 2.5 mg/day, the treatment continues to be stopped.

In some other embodiments, after the patient is treated with herombopag or the pharmaceutically acceptable salt thereof, the patient has a platelet count that is greater than 200 × 10⁹/L and less than or equal to 400 × 10⁹/L, and a daily dose is decreased by 2.5 mg.

In some embodiments, after the patient is treated with herombopag or the pharmaceutically acceptable salt thereof for 14 days, the patient has a platelet count that is greater than 200 × 10⁹/L and less than or equal to 400 × 10⁹/L, and a daily dose is decreased by 2.5 mg.

In another aspect, in some embodiments, after the patient is treated with herombopag or the pharmaceutically acceptable salt thereof, the patient has a platelet count that is greater than or equal to 100 × 10⁹/L and less than 250 × 10⁹/L, and the treatment is maintained at an original dose.

In some embodiments, after the patient is treated with herombopag or the pharmaceutically acceptable salt thereof for 14 days, the patient has a platelet count that is greater than or equal to 100 × 10⁹/L and less than 250 × 10⁹/L, and the treatment is maintained at an original dose.

In some other embodiments, after the patient is treated with herombopag or the pharmaceutically acceptable salt thereof, the patient has a platelet count that is greater than 400 × 10⁹/L, and the treatment is suspended, and when the platelet count decreases to <250 × 10⁹/L, the treatment is resumed, and a daily dose is decreased by 2.5 mg; if a lowest dose used at this time is 2.5 mg/day, the treatment continues to be stopped.

In some embodiments, after the patient is treated with herombopag or the pharmaceutically acceptable salt thereof for 14 days, the patient has a platelet count that is greater than 400 × 10⁹/L, and the treatment is suspended, and when the platelet count decreases to <250 × 10⁹/L, the treatment is resumed, and a daily dose is decreased by 2.5 mg; if a lowest dose used at this time is 2.5 mg/day, the treatment continues to be stopped.

In some other embodiments, after the patient is treated with herombopag or the pharmaceutically acceptable salt thereof, the patient has a platelet count that is greater than 250 × 10⁹/L and less than or equal to 400 × 10⁹/L, and a daily dose is decreased by 2.5 mg.

In some embodiments, after the patient is treated with herombopag or the pharmaceutically acceptable salt thereof for 14 days, the patient has a platelet count that is greater than 250 × 10⁹/L and less than or equal to 400 × 10⁹/L, and a daily dose is decreased by 2.5 mg. In some embodiments, after the patient is treated with herombopag or the pharmaceutically acceptable salt thereof, the patient has a platelet count that is less than <100 × 10⁹/L, and a daily dose is increased by 2.5 mg, up to a maximum of 15 mg/day.

In some embodiments, after the patient is treated with herombopag or the pharmaceutically acceptable salt thereof for 14 days, the patient has a platelet count that is less than <100 × 10⁹/L, and a daily dose is increased by 2.5 mg, up to a maximum of 15 mg/day.

The present disclosure further provides a method for treating chemotherapy-induced thrombocytopenia, the method comprising administering to a patient a therapeutically effective amount of herombopag or a pharmaceutically acceptable salt thereof.

The present disclosure further provides a method for treating chemotherapy-induced thrombocytopenia by using herombopag or a pharmaceutically acceptable salt thereof.

In some embodiments, a starting dose of herombopag or the pharmaceutically acceptable salt thereof is 2.5-10.0 mg, preferably 2.5 mg, 3.75 mg, 5 mg, 7.5 mg, 10 mg, 12.5 mg, or 15 mg.

In some embodiments, a starting dose of herombopag or the pharmaceutically acceptable salt thereof is 7.5 mg.

In some embodiments, in the method for treating chemotherapy-induced thrombocytopenia by using herombopag or a pharmaceutically acceptable salt thereof, an administered dose is 7.5 mg.

In some embodiments, the patient with chemotherapy-induced thrombocytopenia has a platelet count of <75 × 10⁹/L.

In some embodiments, the patient with chemotherapy-induced thrombocytopenia experiences a delay of ≥1 week in chemotherapy relative to an expected time due to thrombocytopenia and has a platelet count of <75 × 10⁹/L.

In some embodiments, herombopag or the pharmaceutically acceptable salt thereof exists as a unit-dose formulation.

In some other embodiments, the unit-dose formulation comprises at least 2.5 mg of herombopag. In some other embodiments, the unit-dose formulation comprises at least 3.75 mg of herombopag. In some embodiments, the unit-dose formulation comprises 5 mg of herombopag. In some embodiments, the unit-dose formulation comprises 7.5 mg of herombopag.

In some embodiments, during the implementation of the methods or uses described in the present disclosure, the platelet count should be regularly monitored, and the dose is adjusted based on the platelet count until a minimum administered dose that maintains a platelet response and achieves effective treatment is reached. Before each administered dose adjustment, it is recommended to maintain the current administered dose for at least 1 week or 2 weeks to clarify its related efficacy.

In some embodiments, the methods of the present disclosure comprise adjusting the dose based on the patient's platelet count after the patient is treated with herombopag or the pharmaceutically acceptable salt thereof for at least 14 days. In some other embodiments, the methods of the present disclosure comprise adjusting the dose based on the patient's platelet count after the patient is treated with herombopag or the pharmaceutically acceptable salt thereof for at least 7 days. In some embodiments, after the patient is treated with herombopag or the pharmaceutically acceptable salt thereof, the patient has a platelet count that is greater than 400 × 10⁹/L, and the treatment is suspended, and when the platelet count decreases to <200 × 10⁹/L, the treatment is resumed, and a daily dose is decreased by 2.5 mg; if a lowest dose used at this time is 2.5 mg/day, the treatment continues to be stopped; preferably after the patient is treated with herombopag or the pharmaceutically acceptable salt thereof for at least 14 days.

In some embodiments, after the patient is treated with herombopag or the pharmaceutically acceptable salt thereof, the patient has a platelet count that is greater than 200 × 10⁹/L and less than or equal to 400 × 10⁹/L, and a daily dose is decreased by 2.5 mg; preferably after the patient is treated with herombopag or the pharmaceutically acceptable salt thereof for at least 14 days.

In some embodiments, after the patient is treated with herombopag or the pharmaceutically acceptable salt thereof, the patient has a platelet count that is greater than or equal to 100 × 10⁹/L and less than 200 × 10⁹/L, and the treatment is maintained at an original dose.

In some other embodiments, after the patient is treated with herombopag or the pharmaceutically acceptable salt thereof, the patient has a platelet count that is greater than 400 × 10⁹/L, and the treatment is suspended, and when the platelet count decreases to <250 × 10⁹/L, the treatment is resumed, and a daily dose is decreased by 2.5 mg; if a lowest dose used at this time is 2.5 mg/day, the treatment continues to be stopped; preferably after the patient is treated with herombopag or the pharmaceutically acceptable salt thereof for at least 14 days.

In some other embodiments, after the patient is treated with herombopag or the pharmaceutically acceptable salt thereof, the patient has a platelet count that is greater than 250 × 10⁹/L and less than or equal to 400 × 10⁹/L, and a daily dose is decreased by 2.5 mg; preferably after the patient is treated with herombopag or the pharmaceutically acceptable salt thereof for at least 14 days.

In some other embodiments, after the patient is treated with herombopag or the pharmaceutically acceptable salt thereof, the patient has a platelet count that is greater than or equal to 100 × 10⁹/L and less than 250 × 10⁹/L, and the treatment is maintained at an original dose. In some embodiments, after the patient is treated with herombopag or the pharmaceutically acceptable salt thereof, the patient has a platelet count that is less than <100 × 10⁹/L, and a daily dose is increased by 2.5 mg, up to a maximum of 15 mg/day; preferably after the patient is treated with herombopag or the pharmaceutically acceptable salt thereof for at least 7 days.

In some embodiments, after the patient is treated with herombopag or the pharmaceutically acceptable salt thereof, when the patient's platelet response rate/trend is expected to cause a delay or a dose reduction in a next chemotherapy cycle, the dose is adjusted upward after 1 week of treatment.

In another aspect, the patient described in the present disclosure does not suffer from a hematopoietic disease other than chemotherapy-induced thrombocytopenia (CIT).

In some embodiments, the other hematopoietic diseases are selected from the group consisting of, but are not limited to, leukemia, primary immune thrombocytopenia, myeloproliferative diseases, multiple myeloma, and myelodysplastic syndrome.

In some embodiments, the method is therapeutically effective during a treatment period.

The unit-dose formulation to which the present disclosure relates generally comprises a pharmaceutically acceptable excipient. The excipient is selected from the group consisting of, but is not limited to, microcrystalline cellulose, lactose, low-substituted hydroxypropylcellulose, and magnesium stearate.

The tumor described in the present disclosure includes, but is not limited to, lung cancer (small cell or non-small cell), breast cancer, and bladder cancer.

As used herein, "treatment period" is generally defined as two consecutive chemotherapy cycles. Chemotherapy cycles are designed based on the half-life of the drugs and the tumor doubling time and can be adjusted depending on the chemotherapeutic drugs.

In some embodiments, the chemotherapy cycle described in the present disclosure is 21 days.

As used herein, "therapeutically effective" is defined as the subject meeting one or all of the following criteria during the treatment period (two consecutive chemotherapy cycles):
1. The subject is able to resume this chemotherapy cycle and achieve a platelet count of ≥100 × 10⁹/L on day 14 after starting treatment with the investigational drug or is able to achieve a platelet count of ≥100 × 10⁹/L within 14 days (inclusive) of starting receiving treatment with the investigational drug.
2. The subject is able to complete two chemotherapy cycles without requiring adjustments to the chemotherapy regimen due to thrombocytopenia (e.g., a delay of ≥4 days in chemotherapy, and/or a reduction of ≥15% in the dose of chemotherapy, and termination of chemotherapy).
3. The subject is able to complete the first chemotherapy cycle (C1) using the same therapy regimen as before enrollment and achieve a platelet count of ≥75 × 10⁹/L on C1D21 (window period: +4 days).
4. No thrombopoietic rescue treatment (platelet transfusion, interleukin-11, or recombinant human thrombopoietin) is used during the treatment period.

The herombopag described in the present disclosure is a compound structure represented by formula I:

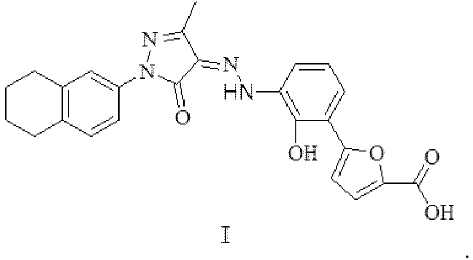

The pharmaceutically acceptable salt of herombopag described in the present disclosure may be a sodium salt, a lithium salt, a potassium salt, a calcium salt, a magnesium salt, an arginine salt, a lysine salt, a methylamine salt, a dimethylamine salt, a trimethylamine salt, an ethylamine salt, a diethylamine salt, a triethylamine salt, an ethanolamine salt, a piperazine salt, a dibenzylethylenediamine salt, a meglumine salt, a tromethamine salt, a tetramethylquaternary ammonium salt, a tetraethylquaternary ammonium salt, or a choline salt, preferably a diethylamine salt, an ethanolamine salt, a choline salt, a piperazine salt, a meglumine salt, or a tromethamine salt, more preferably an ethanolamine salt, and most preferably a diethanolamine salt.

As used herein, "persistent chemotherapy-induced thrombocytopenia" is defined as thrombocytopenia resulting in a delay of ≥1 week in chemotherapy and a platelet count of <75 × 10⁹/L.

In some embodiments, the herombopag or the pharmaceutically acceptable salt thereof is administered at a frequency of once every two days, once daily, twice daily, or three times daily.

According to related research data of the present disclosure, herombopag or the pharmaceutically acceptable salt thereof demonstrates outstanding efficacy in treating persistent chemotherapy-induced thrombocytopenia, significantly increasing the proportion of subjects in which it is therapeutically effective. In addition, the drug has a favorable safety profile. The further development and application of this drug are expected to significantly change the state of treatment of chemotherapy-induced thrombocytopenia.

### DETAILED DESCRIPTION

The present disclosure is further described with reference to the following examples; however, these examples are not intended to limit the scope of the present disclosure.

### Example 1: Study of Efficacy and Safety of Herombopag Olamine Tablets in Treatment of Thrombocytopenia Induced by Chemotherapy for Malignant Tumors

### 1. Study drugs

Investigational drug: herombopag olamine tablets, 2.5 mg/tablet, manufactured and provided by Jiangsu Hengrui Pharmaceuticals Co., Ltd.

Control drug: herombopag olamine simulated tablets (placebo), 2.5 mg/tablet, identical in shape, color, weight, etc. to the herombopag olamine tablets, manufactured and provided by Jiangsu Hengrui Pharmaceuticals Co., Ltd.

### 2. Inclusion criteria

Patients must meet all of the following inclusion criteria to be eligible for the study:
1. Age between 18 years and 75 years (inclusive), regardless of gender.
2. Pathologically or cytologically confirmed diagnosis of malignant tumors, including lung cancer (small cell or non-small cell), breast cancer, bladder cancer, etc.
3. The subject is currently receiving a chemotherapy regimen on a 21-day cycle (excluding other chemotherapy cycle lengths) and requires the use of one or more chemotherapeutic drugs, such as:
   - Antimetabolites, including gemcitabine, etc.;
   - Platinum-based agents, including carboplatin, nedaplatin, cisplatin, lobaplatin, etc.;
   - Anthracyclines, including doxorubicin, daunorubicin, epirubicin, etc.;
   - Alkylating agents, including cyclophosphamide, ifosfamide, etc.
4. The subject experiences a delay of ≥1 week in chemotherapy relative to the expected time due to thrombocytopenia, and the platelet count remains <75 × 10⁹/L.
5. Physical fitness status ECOG score of 0-1.
6. The subject has an expected survival period of ≥12 weeks at the time of screening and is able to receive at least 2 or more cycles of the current chemotherapy regimen.
7. Subjects of childbearing potential must agree to use reliable methods of contraception throughout the study (including male or female condoms, contraceptive foams, contraceptive gels, contraceptive films, contraceptive creams, contraceptive suppositories, abstinence, placement of intrauterine devices, etc.). Female subjects who have undergone a hysterectomy, bilateral salpingectomy, or bilateral tubal ligation or have been postmenopausal for more than 1 year and male subjects who have undergone a bilateral vasectomy or ligation are excluded.
8. The subject is willing to participate in the study, can provide written informed consent, and has good compliance.

Patients meeting any one of the following criteria are excluded from the study:
1. Screening or baseline platelet count of <30 × 10⁹/L.
2. Platelet count of <75 × 10⁹/L prior to the first dose of chemotherapy.
3. Hematopoietic diseases other than chemotherapy-induced thrombocytopenia (CIT), including but not limited to leukemia, primary immune thrombocytopenia, myeloproliferative diseases, multiple myeloma, and myelodysplastic syndrome.
4. Thrombocytopenia due to causes other than CIT within 6 months prior to screening, including but not limited to chronic liver disease, hypersplenism, infections, bleeding, etc.
5. Bone marrow involvement or bone marrow metastasis.
6. Receipt of pelvic or spinal radiotherapy and large-field irradiation of bones within 3 months prior to screening, or currently receiving/planning to receive radiotherapy.
7. History of a severe cardiovascular disease within 6 months prior to screening, such as congestive heart failure (NYHA heart function score of class III-IV), arrhythmias known to increase the risk of thromboembolism, such as atrial fibrillation, post coronary artery stenting, post angioplasty and coronary artery bypass grafting, etc.
8. History of any arterial or venous thromboembolic disease within 6 months prior to screening.
9. Severe bleeding clinical manifestations within 2 weeks prior to screening, such as gastrointestinal or central nervous system bleeding.
10. Brain tumors or brain metastases.
11. In need of urgent treatment, such as for superior vena cava syndrome or spinal cord compression.
12. Absolute neutrophil count of <1.0 × 10⁹/L or hemoglobin < 80 g/L; treatment with granulocyte-colony stimulating factor and red blood cell or EPO transfusions meeting clinical routines is permitted.
13. Significantly abnormal liver function: in patients without liver metastases: ALT/AST > 3ULN (the upper limit of normal) and TBIL > 3ULN; in patients with liver metastases: ALT/AST ≥ 5ULN and TBIL ≥ 5ULN.
14. Abnormal kidney function: serum creatinine ≥ 1.5ULN or eGFR ≤ 60 mL/min (Cockcroft-Gault formula).
15. Receipt of treatment with thrombopoietin receptor agonist drugs (e.g., eltrombopag and romiplostim) or recombinant human thrombopoietin (rhTPO) or recombinant human interleukin-11 (rhIL-11) within 1 month prior to screening.
16. Receipt of platelet transfusions within 3 days prior to randomization/the first dose.
17. Patients known or expected to be allergic to or intolerant of the active ingredient or excipients (including cellulose-lactose, low-substituted hydroxypropylcellulose, and magnesium stearate) in herombopag olamine tablets.
18. The subject is infected with HIV.
19. Women who are pregnant or breastfeeding.
20. Participation in any other study drug or device clinical study within 3 months prior to screening.
21. Other conditions that, in the investigator's opinion, pose a relatively significant risk to the subject's health or safety if the subject participates in the trial, or may impact the assessment of efficacy.

### 3. Route of administration:

Stratification will be performed based on the baseline platelet count (≥50 × 10⁹/L vs. <50 × 10⁹/L) using a stratified block randomization method. Subjects will be randomized in a 1:1 ratio to an investigational group (herombopag olamine tablets, 7.5 mg) or a placebo group (herombopag olamine simulated tablets) to receive corresponding treatment.

### Drug dose:

During the treatment period, subjects randomized to the investigational group will receive treatment with herombopag olamine tablets, with the starting dose being 7.5 mg once daily. After 14 days, the drug dose may be adjusted based on the subject's platelet response or the investigator's judgment. The subjects will orally take the medication on a continuous basis until the completion of 2 chemotherapy cycles. Subjects randomized to the control group will receive treatment with corresponding placebo tablets. During the optional extension period, subjects in the herombopag group may continue to receive treatment, and subjects in the placebo group will receive treatment with herombopag olamine tablets, with the starting dose being 7.5 mg once daily; the drug dose may be adjusted based on the investigator's judgment during the treatment period, and the maximum duration is 4 chemotherapy cycles.

During the optional extension period, subjects in the herombopag group may continue to receive treatment, and subjects in the placebo group will receive treatment with herombopag olamine tablets, with the starting dose being 7.5 mg once daily; the drug dose may be adjusted based on the investigator's judgment during the treatment period, and the maximum duration is 4 chemotherapy cycles.

### Method of administration:

Orally administer once daily on an empty stomach. After orally taking the medication, subjects should refrain from eating for 2 hours to avoid taking it with the meal.

The following products should be used at least 2 hours after taking the medication: dairy products (e.g., milk, yogurt, cheese, and ice cream) or mineral supplements containing multivalent cations (e.g., aluminum, calcium, magnesium, iron, selenium, and zinc).

### Drug adjustment

For each dose adjustment, it is recommended to maintain the dose for 2 weeks to observe its effect. Adjustment rules are as follows:
- When the platelet count is <100 × 10⁹/L, increase the daily dose by 2.5 mg, up to a maximum of 15 mg/day.
- When the platelet count is ≥200 × 10⁹/L and ≤400 × 10⁹/L, decrease the daily dose by 2.5 mg.
- When the platelet count is >400 × 10⁹/L, the treatment can be suspended, and when the platelet count decreases to <200 × 10⁹/L, resume the treatment, and decrease the daily dose by 2.5 mg; if the lowest dose (2.5 mg/day) is used at this time, the treatment can be stopped.
- If the investigator anticipates that the subject's platelet response rate/trend will cause a delay or a dose reduction in the next chemotherapy cycle, the dose can be adjusted upward after 1 week of treatment.

### 4. Clinical endpoints:

### Primary study endpoint:

The primary endpoint is the proportion of subjects in which the drug is therapeutically effective. "Therapeutically effective" is defined as meeting all of the following criteria during the treatment period (two consecutive chemotherapy cycles):
1. The subject is able to resume this chemotherapy cycle and achieve a platelet count of ≥100 × 10⁹/L on day 14 after starting treatment with the investigational drug.
2. The subject is able to complete two chemotherapy cycles without requiring adjustments to the chemotherapy regimen due to thrombocytopenia (e.g., a delay of ≥4 days in chemotherapy, and/or a reduction of ≥15% in the dose of chemotherapy, and termination of chemotherapy).
3. No thrombopoietic rescue treatment (platelet transfusion, interleukin-11, or recombinant human thrombopoietin) is used during the treatment period.

### 5. Study results:

During the study, 60 patients were enrolled, and 59 of them received at least one dose of a study drug and were randomized to the investigational group (n = 28) or the placebo group (n = 31). In the investigational group, 17 patients achieved the primary study endpoint (17/28 [60.7%, 95% CI 40.6 - 78.5]). In the placebo group, 4 patients achieved the primary study endpoint (4/31 [12.9%, 95% CI 3.6 - 29.8]). The OR value was 10.44 [95% CI 2.82-38.65]; p = 0.0001, indicating a significant increase in the number of patients achieving the primary study endpoint in the investigational group.

The incidence of adverse events was comparable between the investigational group and the placebo group. No grade 3 or higher grade treatment-related adverse events or treatment-related serious adverse events were observed in the investigational group.

### Example 2: Study of Efficacy and Safety of Herombopag Olamine Tablets in Treatment of Chemotherapy-Induced Thrombocytopenia

### 1. Study drugs

Investigational drug: herombopag olamine tablets, 2.5 mg/tablet, manufactured and provided by Jiangsu Hengrui Pharmaceuticals Co., Ltd.

Control drug: herombopag olamine simulated tablets (placebo), 2.5 mg/tablet, identical in shape, color, weight, etc. to the herombopag olamine tablets, manufactured and provided by Jiangsu Hengrui Pharmaceuticals Co., Ltd.

### 2. Inclusion criteria

Subjects must meet all of the following criteria to be eligible for the study:
1. Age between 18 years and 75 years (inclusive), regardless of gender.
2. Pathologically or cytologically confirmed diagnosis of malignant tumors, including but not limited to gastric cancer, colorectal cancer, lung cancer (small cell or non-small cell), breast cancer, bladder cancer, etc.
3. The subject is currently receiving chemotherapy on a 21-day cycle (excluding other chemotherapy cycle lengths). The chemotherapy regimen must be a combination therapy regimen containing a platinum-based drug, including a combination therapy regimen with ≥2 chemotherapeutic drugs, or ≥1 chemotherapeutic drug in combination with immunotherapy and (or) targeted therapy, etc.
4. The subject experiences a delay of ≥1 week in chemotherapy relative to the expected time due to CIT and has a platelet count of <75 × 10⁹/L both during the delay and on the day of enrollment.
5. ECOG PS score of 0-1.
6. The subject has an expected survival period of ≥12 weeks and is able to receive at least 2 or more cycles of the current chemotherapy regimen.
7. Female subjects of childbearing potential must have a negative serum pregnancy test within 3 days prior to randomization and must not be breastfeeding. Female subjects of childbearing potential or male subjects whose partners are females of childbearing potential must agree to use acceptable methods of contraception during the study drug administration period and within 30 days of administering the last dose of a study drug.
8. The subject is willing to participate in the study, can provide written informed consent, and has good compliance.

Subjects meeting any one of the following criteria are excluded from the study:
1. Platelet count of <30 × 10⁹/L.
2. Platelet count of <75 × 10⁹/L prior to the first dose of chemotherapy of the current antitumor therapy stage.
3. Hematopoietic diseases, including but not limited to leukemia, primary immune thrombocytopenia, myeloproliferative diseases, multiple myeloma, myelodysplastic syndrome, etc.
4. Diseases causing thrombocytopenia other than CIT within 6 months prior to randomization, including but not limited to chronic liver disease, hypersplenism, infections, etc.
5. Tumor bone marrow involvement or bone marrow metastasis.
6. Prior radiotherapy, particularly to long bones or flat bones (e.g., pelvis and sternum), or currently receiving/planning to receive radiotherapy.
7. Severe clinical symptoms or diseases of the heart within 6 months prior to randomization, such as New York Heart Association (NYHA) class 3 or higher class heart failure, unstable angina pectoris, myocardial infarction, and clinically significant supraventricular or ventricular arrhythmias requiring treatment or intervention.
8. The subject has thrombophilia or is currently receiving thrombolytic/anticoagulant therapy.
9. Arterial or venous thromboembolic events within 6 months prior to randomization, including but not limited to cerebrovascular accidents (e.g., transient ischemic attacks and cerebral infarction), deep vein thrombosis (excluding intramuscular vein thrombosis not requiring treatment), pulmonary embolism, etc.
10. Clinically significant bleeding symptoms or definite bleeding diathesis clinical manifestations within 2 weeks prior to randomization, such as digestive tract or central nervous system bleeding.
11. Brain tumors or tumor brain metastases.
12. Complications requiring urgent treatment, such as superior vena cava syndrome and spinal cord compression.
13. Absolute neutrophil count (ANC) of <1.0 × 10⁹/L or hemoglobin (Hb) < 80 g/L (treatment with red blood cell, erythropoietin [EPO], and recombinant human granulocyte-colony stimulating factor [G-CSF] transfusions meeting clinical routines is permitted).
14. Significantly abnormal liver function: aspartate aminotransferase (AST) and alanine aminotransferase (ALT) > 3× ULN (the upper limit of normal) and total bilirubin (TBIL) > 1.5× ULN (enrollment of those with liver metastases, ALT and AST ≤ 5× ULN, and TBIL ≤ 3× ULN is permitted).
15. Abnormal kidney function: serum creatinine (Cr) ≥ 1.5× ULN or creatinine clearance (CrCL) ≤ 50 mL/min (CockcroftGault formula).
16. Receipt of treatment with rhIL-11, rhTPO, and thrombopoietin receptor agonist drugs (e.g., eltrombopag and romiplostim) within 28 days prior to randomization; receipt of treatment with other drugs that affect platelet function (e.g., aspirin, caffeic acid tablets, non-steroidal anti-inflammatory drugs, and leucogen tablets) within 7 days prior to randomization; or receipt of platelet transfusions within 3 days prior to randomization.
17. Known or expected allergy to or intolerance of the active ingredient or excipients in herombopag olamine tablets.
18. Known history of positive human immunodeficiency virus (HIV) testing or acquired immunodeficiency syndrome (AIDS).
19. Participation in any other study drug or device clinical study within 28 days prior to randomization.
20. Other factors that, in the investigator's opinion, may impact study results or lead to premature termination of the study, such as alcohol abuse, drug abuse, substance abuse, and other severe diseases (including psychiatric diseases) that require concomitant treatment; or significant laboratory test abnormalities, and factors (e.g., familial or social) that could impact medication safety.

### 3. Route of administration:

Orally administer herombopag olamine tablets/erombopag olamine simulated tablets once daily on an empty stomach. After orally taking the medication, subjects should refrain from eating for 2 hours to avoid taking it with the meal. The following products should be used at least 2 hours after taking the medication: dairy products (e.g., milk, yogurt, cheese, and ice cream) or mineral supplements containing multivalent cations (e.g., aluminum, calcium, magnesium, iron, selenium, and zinc).

Dose suspensions and adjustments according to protocol requirements are permitted.

### Drug dose

This trial includes investigational group 1, investigational group 2, and investigational group 3 (control group).

During the correction treatment period, subjects randomized to investigational group 1 and investigational group 2 will receive treatment with herombopag olamine tablets, with the starting dose being 7.5 mg once daily (oral administration on a continuous basis), and subjects randomized to the control group will receive treatment with corresponding doses of herombopag olamine simulated tablets. During the maintenance treatment period, subjects randomized to investigational group 1 will continue to receive treatment with herombopag olamine tablets, and subjects randomized to investigational group 2 and the control group will receive treatment with corresponding doses of herombopag olamine simulated tablets. During the extension treatment period, subjects randomized to investigational group 1 will continue to receive treatment with herombopag olamine tablets; meanwhile, subjects randomized to investigational group 2 and the control group will also receive treatment with herombopag olamine tablets, with the starting dose being 7.5 mg once daily (oral administration on a continuous basis) and the maximum duration being 2 chemotherapy cycles. Subjects must enter the correction treatment period and start receiving continuous treatment with a study drug within 1 natural day post-randomization. During the correction treatment period, if a subject's platelet count recovers to ≥100 × 10⁹/L within 14 days of treatment with a study drug, they should use the same combination therapy regimen as before enrollment, enter the maintenance treatment period, and resume chemotherapy in time. If a subject's platelet count remains <100 × 10⁹/L after 14 days of treatment with a study drug and they have not started the first chemotherapy cycle, it is permitted to perform thrombopoietic rescue treatment and (or) resume chemotherapy in due time based on the investigator's judgment.

After entering the maintenance treatment period, subjects must use the same combination therapy regimen as before enrollment (including the same dose intensity, administration regimen, etc.) and complete 2 consecutive chemotherapy cycles (with the exception of second cycle therapy regimen adjustments due to CIT). Meanwhile, subjects randomized to investigational group 1 and the control group will continue to receive treatment with herombopag olamine tablets or herombopag olamine simulated tablets, and subjects randomized to investigational group 2 will stop receiving treatment with herombopag olamine tablets and orally take herombopag olamine simulated tablets on a continuous basis. Before the second chemotherapy cycle of the maintenance treatment period, the subject's platelet count must be ≥100 × 10⁹/L; if the second chemotherapy cycle has been delayed for ≥4 days due to thrombocytopenia, it is permitted to resume chemotherapy in due time based on the investigator's judgment. During the maintenance treatment period, based on changes in the subjects' platelet counts, the investigator will adjust the study drug dose and (or) perform thrombopoietic rescue treatment according to the study drug dose adjustment rules and the thrombopoietic rescue treatment rules.

After the maintenance treatment period, some subjects may, based on the investigator's judgment, enter the extension treatment period and receive a maximum of 2 chemotherapy cycles. After entering the extension treatment period, subjects randomized to investigational group 1 will continue to receive treatment with herombopag olamine tablets, and subjects randomized to investigational group 2 and the control group will also start receiving treatment with herombopag olamine tablets, with the starting dose being 7.5 mg once daily (oral administration on a continuous basis) and the maximum duration being 2 chemotherapy cycles. In addition, all subjects entering the extension treatment period will undergo case-by-case unblinding, and based on changes in the subjects' platelet counts, the investigator will adjust the study drug dose and (or) perform thrombopoietic rescue treatment according to the study drug dose adjustment rules and the thrombopoietic rescue treatment rules.

### Dose adjustment

During the study, the platelet count should be regularly monitored, and the dose is adjusted based on the platelet count until a minimum administered dose that maintains a platelet response and achieves effective treatment is reached. Before each administered dose adjustment, it is recommended to maintain the current administered dose for at least 2 weeks to clarify its related efficacy. Specific adjustment rules are as follows:
- If the platelet count is <100 × 10⁹/L: adjust the dose upward by one level (with 2.5 mg as a unit) based on the current administered dose, up to a maximum of 15 mg/day (If the investigator anticipates that the subject's platelet response rate/trend will cause a delay or a chemotherapeutic drug dose intensity reduction in the next chemotherapy cycle, the dose can be adjusted upward by one level after 1 week of treatment with the current administered dose).
- If the platelet count is ≥100 ×10⁹/L and <250 × 10⁹/L, maintain the current administered dose.
- If the platelet count is ≥250 ×10⁹/L and ≤400 × 10⁹/L, adjust the dose downward by one level (with 2.5 mg as a unit) based on the current administered dose.
- If the platelet count is >400 × 10⁹/L, the treatment can be suspended, and when the platelet count decreases to <250 × 10⁹/L, adjust the dose downward by one level (with 2.5 mg as a unit) based on the administered dose before suspension and resume the treatment.

### 4. Clinical endpoints:

**Primary study endpoint:** the proportion of subjects in which the drug is therapeutically effective.

"Therapeutically effective" is defined as the subject meeting all of the following criteria after starting receiving treatment with a study drug:
1. The subject is able to achieve a platelet count of ≥100 × 10⁹/L within 14 days (inclusive) of treatment with a study drug.
2. The subject is able to complete the first chemotherapy cycle (C1) using the same therapy regimen as before enrollment and achieve a platelet count of ≥75 × 10⁹/L on C1D21 (window period: +4 days).
3. After starting receiving treatment with a study drug and until C1D21 (window period: +4 days), no thrombopoietic rescue treatment (e.g., platelet transfusions or administration of platelet growth promoting drugs including recombinant human interleukin-11, recombinant human thrombopoietin, etc. for injection) is received.

**Key secondary study endpoint:** the proportion of subjects who have a platelet count of ≥100 × 10⁹/L at the start of the first chemotherapy cycle (C1D1 [window period: -1 day]) and also meet the following criteria, i.e., the therapeutically effective proportion during the maintenance treatment period:
1. The subject is able to complete the first chemotherapy cycle (C1) using the same therapy regimen as before enrollment and achieve a platelet count of ≥100 × 10⁹/L on C1D21 (window period: +4 days) and does not experience a therapy regimen adjustment due to thrombocytopenia (e.g., a delay of ≥4 days in chemotherapy, a reduction of ≥15% in chemotherapy dose intensity, and premature termination of chemotherapy) in the second chemotherapy cycle (C2).
2. After starting receiving treatment with a study drug and until C1D21 (window period: +4 days), no thrombopoietic rescue treatment (e.g., platelet transfusions or administration of platelet growth promoting drugs such as recombinant human thrombopoietin and recombinant human interleukin-11 for injection) is received.

### Secondary efficacy study endpoints:

1. The time when the blood platelet count is ≥100 × 10⁹/L for the first time after treatment with a study drug is received.
2. The proportion of subjects achieving a platelet count of ≥100 × 10⁹/L within 14 days of receiving treatment with a study drug.
3. The proportion of subjects starting the first chemotherapy cycle (C1) within 14 days of receiving treatment with a study drug.
4. The proportion of subjects who are able to complete the first chemotherapy cycle (C1) using the same therapy regimen as before enrollment and achieve a platelet count of ≥75 × 10⁹/L on C1D21 (window period: +4 days).
5. The proportion of subjects who are able to complete the first chemotherapy cycle (C1) using the same therapy regimen as before enrollment and achieve a platelet count of ≥100 × 10⁹/L on C1D21 (window period: +4 days).
6. The proportion of subjects who are able to complete two consecutive chemotherapy cycles (C1 and C2) using the same therapy regimen as before enrollment and do not experience a therapy regimen adjustment due to thrombocytopenia (e.g., a delay of ≥4 days in chemotherapy, a reduction of ≥15% in chemotherapy dose intensity, and premature termination of chemotherapy) during the cycles.
7. The proportion of subjects who are able to complete two consecutive chemotherapy cycles (C1 and C2) using the same therapy regimen as before enrollment and achieve a platelet count of ≥75 × 10⁹/L on C2D21 (window period: +4 days).
8. The proportion of subjects receiving at least 1 thrombopoietic rescue treatment as specified in the protocol after starting receiving treatment with a study drug.
9. Subjects' platelet counts at various time points of visits after starting receiving treatment with a study drug.

## Claims

1. Use of herombopag or a pharmaceutically acceptable salt thereof in the preparation of a medicament for treating chemotherapy-induced thrombocytopenia, wherein the herombopag or the pharmaceutically acceptable salt thereof is a unit-dose formulation comprising 7.5 mg of herombopag.

2. The use according to claim 1, wherein a patient with chemotherapy-induced thrombocytopenia has a platelet count of <75 × 10⁹/L.

3. The use according to claim 1 or 2, wherein the patient with chemotherapy-induced thrombocytopenia experiences a delay of ≥1 week in chemotherapy relative to an expected time due to thrombocytopenia and has a platelet count of <75 × 10⁹/L.

4. The use according to any one of claims 1-3, wherein after the patient is treated with herombopag or the pharmaceutically acceptable salt thereof, the patient has a platelet count that is greater than or equal to 100 × 10⁹/L and less than 250 × 10⁹/L, and the treatment is maintained at an original dose.

5. The use according to any one of claims 1-3, wherein after the patient is treated with herombopag or the pharmaceutically acceptable salt thereof, the patient has a platelet count that is greater than 400 × 10⁹/L, and the treatment is suspended, and when the platelet count decreases to <250 × 10⁹/L, the treatment is resumed, and a daily dose is decreased by 2.5 mg; if a lowest dose used at this time is 2.5 mg/day, the treatment continues to be stopped.

6. The use according to any one of claims 1-3, wherein after the patient is treated with herombopag or the pharmaceutically acceptable salt thereof, the patient has a platelet count that is greater than 250 × 10⁹/L and less than or equal to 400 × 10⁹/L, and a daily dose is decreased by 2.5 mg.

7. The use according to any one of claims 1-3, wherein after the patient is treated with herombopag or the pharmaceutically acceptable salt thereof, the patient has a platelet count that is less than <100 × 10⁹/L, and a daily dose is increased by 2.5 mg, up to a maximum of 15 mg/day.

8. The use according to any one of claims 1-7, wherein a dose at which herombopag or the pharmaceutically acceptable salt thereof is administered is adjusted based on the platelet count; when the patient's platelet response rate/trend is expected to cause a delay or a dose reduction in a next chemotherapy cycle, the dose is adjusted upward after 1 week of medication.

9. The use according to any one of claims 1-8, wherein the use is therapeutically effective during a treatment period.
